(19) Europäisches Patentamt
European Patent Office
Office européen des brevets

(11) EP 0 597 900 B1

(12) **EUROPEAN PATENT SPECIFICATION**

(45) Date of publication and mention
of the grant of the patent:
**07.01.1998 Bulletin 1998/02**

(21) Application number: **92915842.6**

(22) Date of filing: **16.07.1992**

(51) Int Cl.6: **C09B 67/36**, C09B 45/01,
G01N 21/03, G01N 21/78,
G01N 33/48, G01N 33/49,
C09B 29/00, G01N 31/22,
G01N 33/84

(86) International application number:
**PCT/AU92/00359**

(87) International publication number:
**WO 93/03100 (18.02.1993 Gazette 1993/05)**

(54) **COLORIMETRIC DETERMINATION OF MAGNESIUM IONS IN FLUIDS**

Kolorimetrische Bestimmung von Magnesiumionen in Flüssigkeiten

DOSAGE PAR COLORIMETRIE D'IONS DE MAGNESIUM DANS DES FLUIDES

(84) Designated Contracting States:
**DE ES FR GB GR IT**

(30) Priority: **26.07.1991 AU 7466/91**

(43) Date of publication of application:
**25.05.1994 Bulletin 1994/21**

(73) Proprietor: **SEBA DIAGNOSTICS PTY. LTD.
Burnley, VIC 3121 (AU)**

(72) Inventors:
• **BALAZS, Nicholas, Dennis, Henry
Burnley, VIC 3121 (AU)**
• **SECOMBE, John, William
Elwood, VIC 3184 (AU)**

(74) Representative: **Tubby, David George et al
MARKS & CLERK,
57-60 Lincoln's Inn Fields
London WC2A 3LS (GB)**

(56) References cited:
**EP-A- 0 254 202        US-A- 4 166 093
US-A- 5 057 435**

• **PATENT ABSTRACTS OF JAPAN vol. 7, no. 274
(P-241)(1419) 7 December 1983 & JP-A-58 153
166 (FUJIHIRA KOGYO K. K.) 12 September 1983**
• **DERWENT ABSTRACT Accession No. 21774,
Class E31; & SU,A,833542 (GIREMET RARE
MET) 21 September 1979 (21.09.79).**
• **DERWENT ABSTRACT Accession No. 15311,
Class E33; & SU,A,827393 (GIREMET RARE
METAL) 25 July 1977 (25.07.77).**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

This invention relates to the colorimetric determination of magnesium ions in fluids, particularly in biological fluids such as blood serum, blood plasma, urine and the like. In particular, the invention relates to a colorimetric method for the quantitative analysis of the concentration of magnesium ions in such fluids.

Magnesium is quantitatively the second most abundant intracellular cation and is the fourth most abundant cation overall in the body. The mechanism of magnesium control within the body has not been fully elucidated however the narrow limits of normal plasma magnesium (0.7 to 1.0 mmol/l) imply close homeostatic control. Magnesium measurements in biological fluids are relevant in both magnesium deficiency (hypomagnesaemia) and magnesium excess (hypermagnesaemia).

Hypomagnesaemia can result from:

(i) abnormal loss eg. diarrhoea, malabsorption, renal tubular acidosis, diuretic therapy, hyperparathyroidism etc; or
(ii) reduced intake eg. severe prolonged malnutrition.

Hypermagnesaemia can result from:

(i) acute or chronic renal failure;
(ii) haemodialysis against a fluid with a high magnesium content (hard water); or
(iii) adrenocortical hypofunction.

Plasma (or serum) and urine magnesium estimations are a useful indicator of total body magnesium homeostasis.

Modern methods for the assay of magnesium ions include both atomic absorption spectrophotometry and colorimetric dye-complexing methods. Atomic absorption spectrophotometry is perhaps the best method for the determination of magnesium ions in fluids, however this method requires expensive instrumentation. More recent methods for magnesium determination have been colorimetric dye-complexing methods which rely on the formation of a coloured complex of magnesium ions with a metallochromic dye.

In general, colorimetric methods usually involve the addition of an aqueous dye-containing reagent to the sample fluid resulting in the formation of a coloured magnesium-dye complex having a characteristic wavelength of maximum light absorption. By exposing the sample of the fluid containing the complexed dye reagent to a light source of the characteristic wavelength, and thereafter measuring the degree of light absorption, the content of magnesium ions in the sample fluid can be simply determined from a graph constructed from absorbance measurements made on standard solutions containing known concentrations of magnesium ions.

In Australia, the most popular colorimetric method for the determination of magnesium ions at present is a method involving the metallochromic dye, Calmagite which is used in about 50% of magnesium determinations. A further 20% of determinations use the dye Magon, whilst about 12% of determinations are performed using atomic absorption spectrophotometry. The remainder of determinations are performed using other miscellaneous colorimetric dye-complexing procedures, including those using such dyes as Eriochrome Black P and Methylthymol Blue.

One of the major difficulties which is encountered with magnesium determination using the dye Magon (Xylidyl Blue) is that citrate interferes with the reaction of magnesium ions with the dye. The result is that sample fluids containing citrate such as citrated-plasma specimens or specimens from patients who have recently received intravenous solutions containing citrate, including blood transfusions, are precluded from analysis due to inaccurate results.

In the most popular colorimetric method using the metallochromic dye, Calmagite, [1-(1 -hydroxy-4-methyl-2-phenylazo)-2-naphthol-4-sulfonic acid], the working reagent containing Calmagite, bound to a mixture of the 9-ethyleneoxide adduct of p-nonylphenyl(Bion NE-9) and polyvinylpyrrolidone (Bion PVP), is added to a sample such as a serum sample and the magnesium ions in the sample cause the formation of a pink magnesium-Calmagite complex which is measured at 532nm. The presence of polyvinylpyrrolidone and the 9-ethyleneoxide adduct of p-nonylphenyl prevents interaction of protein in biological samples with the magnesium-Calmagite complex to influence the absorption spectrum of the complex which results in diminished accuracy of the measured magnesium ion concentration (see U.S. patent No. 3,754,864 to Gindler). The working reagent used in the Calmagite method also contains EGTA [ethylenebis (oxyethylenenitrilo)tetraacetic acid] to prevent calcium interference by preferential chelation of calcium ions, as well as potassium cyanide to prevent interference of heavy metals by formation of alternative complexes.

It is an object of the present invention to provide an alternative method for the accurate quantitative determination of magnesium ions in complex biological and aqueous sample fluids by a spectrophotometric method using a metallochromic dye, despite the presence of other potentially interfering substances in the sample fluid.

In accordance with the present invention, there is provided a method for the colorimetric determination of magnesium ions in a fluid which comprises the step of contacting a sample of said fluid with a reagent comprising the metallochromic dye substance Arsenazo I [2-(4,5-dihydroxy-2,7-disulpho-3-naphthylazo)benzene-arsonic acid] together with a selective complexing agent, and determining the formation of a magnesium-dye complex.

Preferably, the step of determining the formation of a magnesium-dye complex is carried out by measurement of light absorption at 550-580nm, preferably at or near 568nm. The absorbance measurements obtained with the sample fluid are then compared with corresponding measurements made on standard solutions as in the previously known colorimetric methods.

In another aspect, the present invention also provides a reagent for the colorimetric determination of magnesium ions in fluids which comprises, in combination, the metallochromic dye substance, Arsenazo I together with a selective complexing agent.

Preferably the reagent has a pH in the range of 7.5 to 9.5, more preferably in the range of 8.5 to 8.8.

To the knowledge of the present inventors, Arsenazo I dye has not previously been used for the determination of magnesium ions in fluids as other cations, notably calcium ions, give a positive interference resulting in erroneous over-estimation of true magnesium ion concentration in complex biological fluids such as serum, plasma and urine. As broadly described above, the reagent used in accordance with the present invention includes a selective complexing agent which removes such interfering entities and permits the use of Arsenazo I dye to accurately measure magnesium ions free of interference.

The selective complexing agent which is used in the method and reagent of the present invention is preferably a highly selective complexing agent, most preferably BAPTA[1,2-bis(2-aminophenoxy)ethane-N,N,N$^1$,N$^1$-tetraacetic acid] which has been found to be particularly effective in removing potentially interfering substances. Other selective complexing agents which may be used in accordance with the present invention include EGTA (as used in the Calmagite method disclosed above). Whilst EGTA is inferior to BAPTA in that it has some affinity for magnesium, this problem can be overcome where EGTA is used as the selective complexing agent by addition of strontium ions to the reagent mixture. Strontium ions have a higher affinity for EGTA than magnesium ions but less affinity than calcium ions. As a result, calcium ions present in the sample being analysed will displace strontium ion and bind to the EGTA whereas magnesium ions will not, so that the magnesium ions in the sample will then be accurately analysed by complex formation with the Arsenazo I dye. It should be noted in this regard that where EGTA is used as the selective complexing agent together with strontium ions, the displaced strontium ions do not themselves interfere with the magnesium-dye complex formation.

As described above, the reagent used in the method of the present invention has a pH in the range 7.5 to 9.5, more preferably 8.5 to 8.7. Most preferably the reagent is buffered to a mild alkaline pH (8.8) using a buffer which does not bind metal ions. A particularly suitable buffer is tris(hydroxymethyl)amino methane (or Tris).

Preferably also, the reagent contains a detergent such as to solubilise protein where present in the sample fluid, particularly biological fluids such as serum, plasma and urine. Particularly suitable detergents include amphoteric detergents such as Empigen BB-AU, however other detergents which have been tested and found suitable for use in the reagent of the invention include Triton X-100, Triton X-405 and Brij 35. To date, however Empigen BB-AU is the surfactant/detergent which has proved to be the most efficient protein solubiliser.

Finally, the reagent used in the method of this invention may include other components which play no part in the reaction, for example sodium azide may be added as a preservative against fungal or bacterial growth in the reagent.

The method and reagent of the present invention can provide significant advantages in the determination of magnesium ions when compared with the Calmagite method described in detail above. In particular, whereas the Calmagite working reagent is usually made up from a kit supplying two separate reagents, namely a dye reagent (containing Calmagite, Bion NE-9 and Bio PVP) and a base reagent (containing potassium cyanide, potassium hydroxide and EGTA), the reagent of the present invention can be supplied as a single reagent buffered to a mild alkaline pH, which has an excellent closed container stability (greater than 2 years) at ambient temperatures. Refrigeration is not required. Furthermore, the reagent of the present invention does not require the addition of components such as polyvinylpyrrolidone and the 9-ethylene oxide adduct of p-nonylphenol to prevent interference by proteins from the sample fluid. The method and reagent of the present invention also provide very rapid (almost instant) colour formation with the chromogenic magnesium-dye complex product being stable for an extended period (in excess of 12 hours). The method is also accurate and excellent agreement is obtained when compared with the results obtained using atomic absorption spectrophotometry as a reference method. Finally, the method and reagent of the present invention provide for accurate measurement without interference from calcium ions even at grossly elevated calcium concentrations (greater than 6 mmol/l).

While the foregoing description of the method and reagent of this invention refers most particularly to "wet" systems utilising a reagent in liquid form, the reagent of this invention may also be used in "dry" analytical systems in which the reagent is deposited on or impregnated into a solid support or carrier, for example a plastics material film or a porous solid support. In such "dry" systems, the water content of the sample being analysed rehydrates the reagent and the color change resulting from formation of the magnesium - dye complex proceeds on the solid support or carrier. Formation of the magnesium - dye complex is then determined either by absorbance (in the case of a transparent support or carrier) or reflectance (in the case of an opaque support or carrier).

Further details of the method and reagent of the present invention, and of the accuracy and stability of the method of the present invention for the colorimetric determination of magnesium ions in sample fluids, are disclosed in the following examples and in the accompanying drawings. It should be noted that these examples are included by way of illustration of the present invention, and not by way of limitation.

**Example 1**

(a) Reagent of the present invention
A preferred reagent is an aqueous composition comprising:

| | |
|---|---|
| 0.080 g/L | Arsenazo I [2-(4,5-dihydroxy-2,7-disulpho-3-naphthylazo)benzene-arsonic acid] |
| 0.125 g/L | BAPTA [1,2-bis(2-aminophenoxy)ethene- N,N,N,'N'-tetraacetic acid] |
| 0.5 ml/L | Empigen BB-AU |
| 12 g/L | Tris |
| 1 g/L | Sodium Azide. |

The pH of this solution is adjusted to 8.7 - 8.8 with hydrochloric acid.

(b) Experimental procedure for determination of magnesium ions. Using suitably accurate and precise pipetting devices, 25 microlitres of magnesium ion calibration solution or sample to be analysed are added to tubes containing 1.50 ml. of the reagent of the present invention as described in (a) above. Each tube is mixed thoroughly and the absorbance of each tube read at 568 nm against a blank tube containing 1.50 ml of reagent and 25 microlitres of pure water.
The magnesium ion concentration of the sample (unknown) is calculated as follows:

$$\text{Mg. conc. of unknown} = \frac{[\text{Mg. conc. of calibrator}]}{\text{Absorb. of calibrator}} \times \text{Absorb. of unknown}$$

**Example 2        Comparison with atomic absorption spectrophotometry (AAS).**

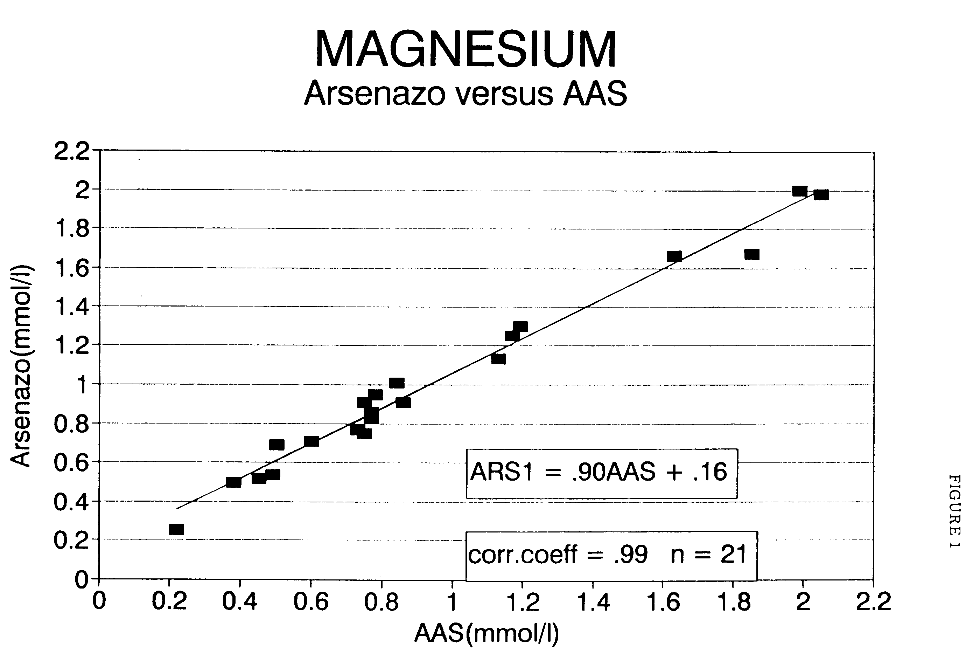

Twenty-one randomly selected patient specimens (serum and plasma) were analysed in duplicate using the reagent and experimental procedure of Example 1 and a Roche Cobas Bio automated analyser, and by atomic adsorption spectroscopy (AAS) performed independently by a private consulting laboratory. The results are set out in Figure 1. Both set of results were obtained using a primary aqueous magnesium calibrator of 1.00 mmol/l concentration.

**Example 3        Effect of added calcium.**

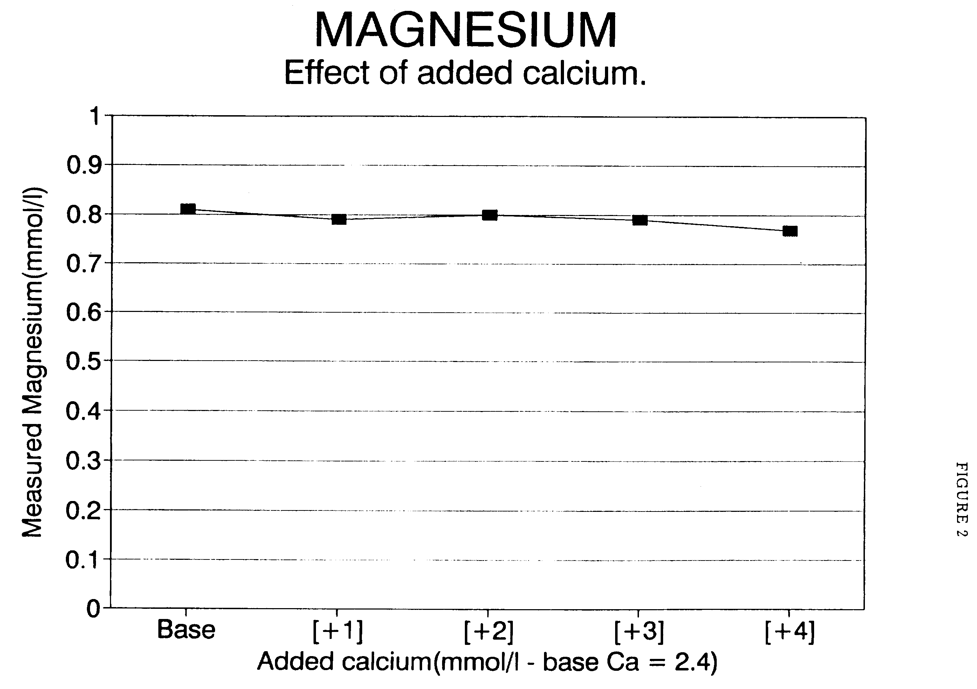

Increasing amounts of a concentrated aqueous calcium solution (50 mmol/l) were added to a human serum pool (magnesium 0.80 mmol/l, calcium 2.40 mmol/l) to give a final calcium concentration of 6.4 mmol/l. The magnesium levels in this series were then assayed in accordance with Example 1, and demonstrated no interference from the added calcium. The results are shown in Figure 2.

**Example 4        Reagent stability.**

Most colorimetric magnesium determination methods use highly alkaline reaction mixtures. These have a major disadvantage in that they are relatively unstable, particularly in "open" vessels as used on several automated analysers. In these systems, and to a lesser extent in closed systems the alkaline reagent absorbs atmospheric carbon dioxide which leads to a pH change in the reagent, altering performance characteristics and requiring constant recalibration of the assay.

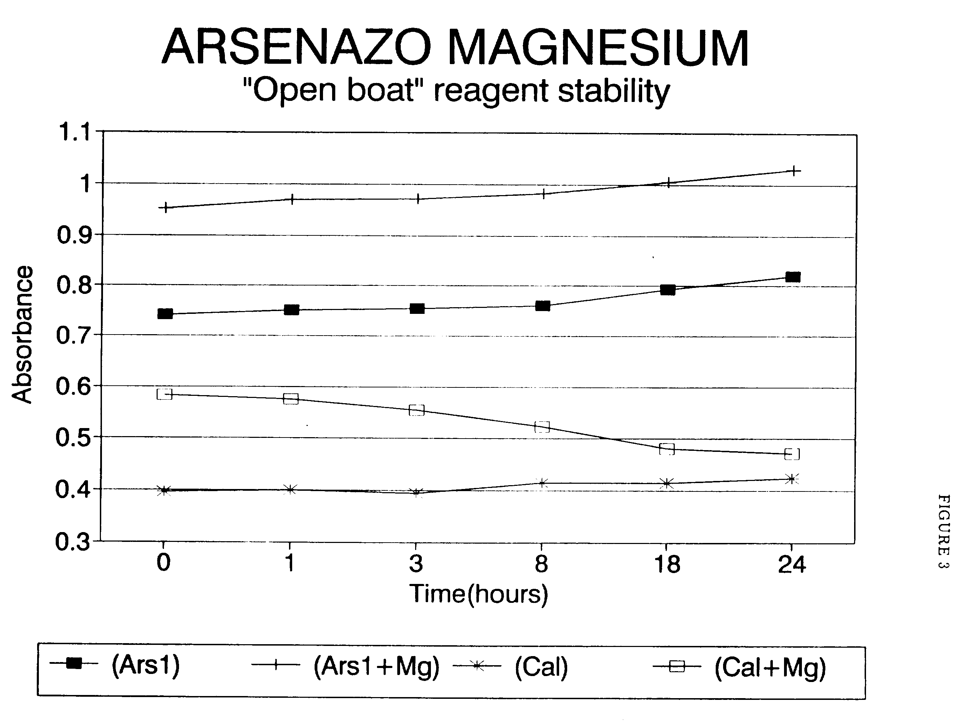

Figure 3 shows data from solutions of the reagent of Example 1 with and without magnesium, and Calmagite reagent with and without magnesium. The solutions were left in open vessels and their respective absorbances monitored over a 24 hour period. As can be seen, the absorbances in the Calmagite solutions are not stable, whereas a slight increase in absorbance is shown with the present reagent, believed to be due to evaporation only.

**Example 5        Absorbance versus time.**

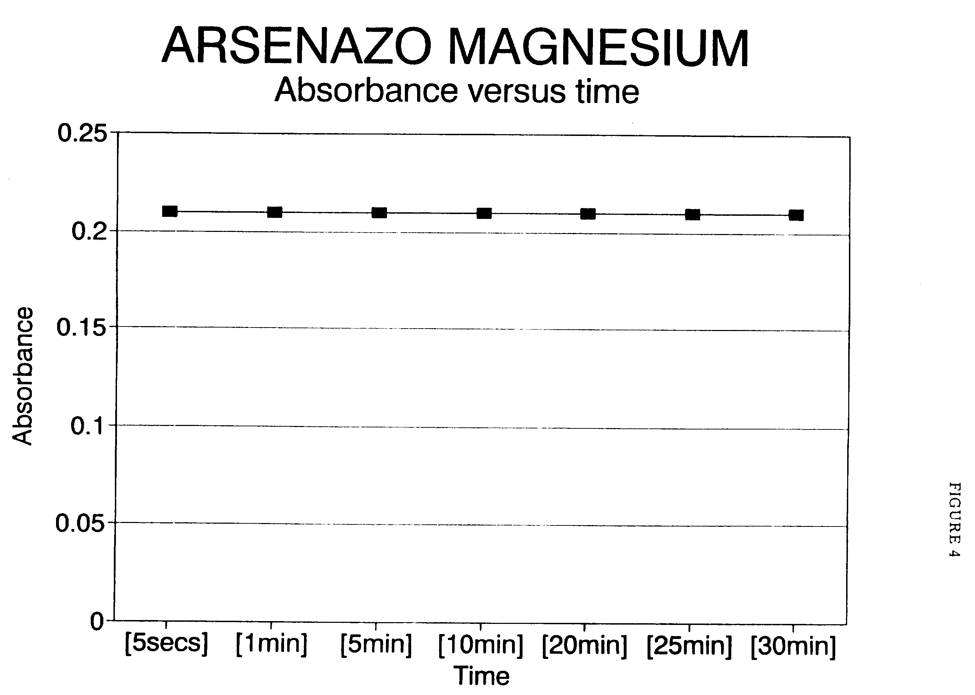

Human serum was mixed with the reagent of Example 1 and the absorbance monitored over 30 minutes. The results are shown in Figure 4. The earliest possible reading on the spectrophotometer in use was 5 seconds, and the results show that there was no detectable absorbance change from 5 seconds through to 30 minutes. Most automated instruments have an ideal measuring "window" of time from about 1 minute through to 15 minutes depending on the instrument.

### Example 6      Magnesium recovery.

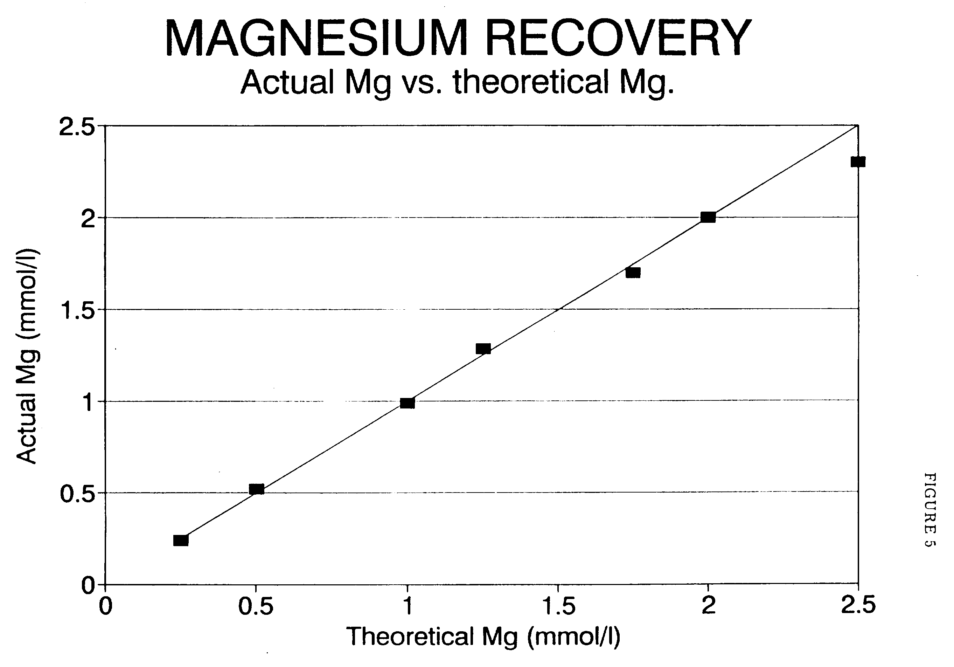

Increasing amounts of a concentrated magnesium solution were added to a human serum with a low magnesium value (0.25 mmol/l). Theoretical magnesium concentration of the "spiked" solutions is plotted in Figure 5 against the values recovered when the solutions were measured with the reagent of Example 1. Recovery was in the range of 98 to 101% up to 2 mmol/l.

### Example 7      Absorbance versus concentration.

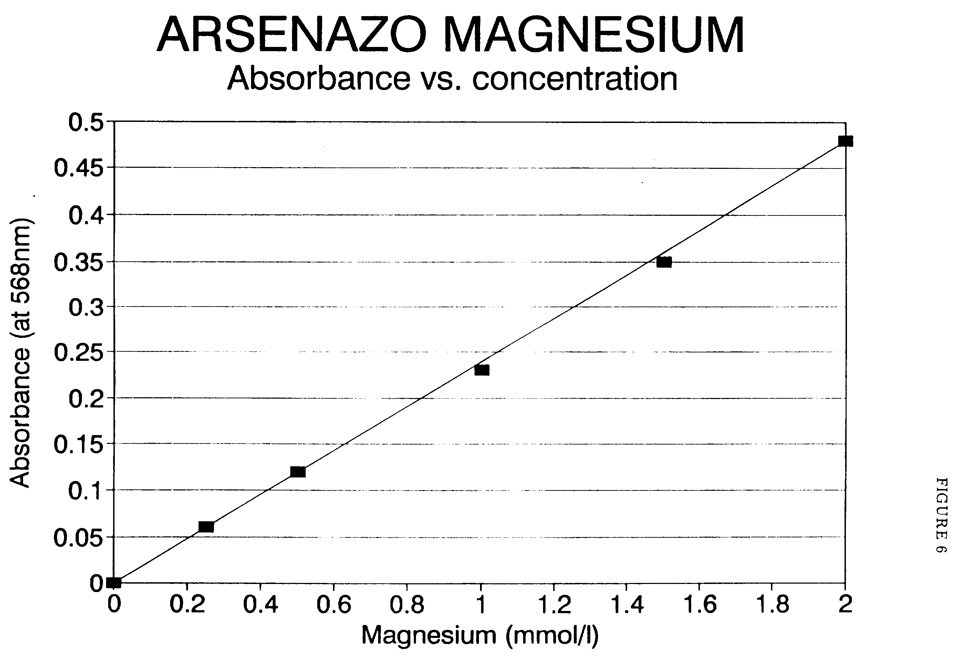

Aqueous primary magnesium calibration material was prepared by diluting a commercial AAS magnesium calibrator, from 0.25 to 2.00 mmol/l. These calibrators were analysed with the reagent of Example 1 and absorbance versus concentration plotted in Figure 6. Linearity to 2.0 mmol/l was demonstrated.

Those skilled in the art will appreciate that the invention described herein is susceptible to variations and modifications other than those specifically described. It is to be understood that the invention includes all such variations and modifications which fall within its spirit and scope. The invention also includes all of the steps, features, compositions and compounds referred to or indicated in this specification, individually or collectively, and any and all combinations of any two or more of said steps or features.

## Claims

1. A method for the colorimetric determination of magnesium ions in a fluid, which comprises the step of contacting a sample of said fluid with a reagent comprising the metallochromic dye substance, 2-(4,5-dihydroxy-2,7-disulpho-3-naphthylazo)benzene-arsonic acid [Arsenoazo I], together with a selective complexing agent, and determining the formation of a magnesium-dye complex

2. A method according to claim 1, wherein said determination of the formation of a magnesium-dye complex is carried out by measurement of light absorption of 550-580nm.

3. A method according to claim 2, wherein said determination is carried out by measurement of light absorption at or near 568nm.

4. A method according to any of claims 1 to 3, wherein said selective complexing agent removes interfering cations, particularly calcium ions, from said fluid sample.

5. A method according to claim 4, wherein said selective complexing agent is 1,2-bis(2-aminophenoxy)ethane-N,N, N',N'-tetracetic acid [BAPTA].

6. A method according to claim 4, wherein said selective complexing agent is ethylenebis(oxyethylenenitrilo)tetraacetic acid [EGTA].

7. A method according to claim 6, wherein said reagent further comprises strontium ions.

8. A method according to any of claims 1 to 7, wherein said reagent is buffered to a pH in the range 7.5 to 9.5, preferably 8.5 to 8.7.

9. A method according to claim 8, wherein said reagent comprises the buffer tris(hydroxymethyl)amino methane [TRIS].

10. A method according to any of claims 1 to 9, wherein said reagent further comprises a detergent to solubilise any protein present in said fluid sample.

11. A method according to claim 10 wherein said detergent is an amphoteric detergent, preferably Empigen BB-AU.

**12.** A method according to claim 10 wherein said detergent is selected from Triton X-100, Triton X-405 and Brij 35.

**13.** A reagent for the colorimetric determination of magnesium ions in fluids which comprises in combination, the metallochromic dye substance, 2-(4,5-dihydroxy-2,7-disulpho-3-naphthylazo)benzene-arsonic acid [Arsenoazo I], together with a selective complexing agent.

**14.** A reagent according to claim 13, wherein said selective complexing agent removes interfering cations particularly calcium ions.

**15.** A reagent according to claim 14, wherein said selective complexing agent is 1,2-bis(2-aminophenoxy)ethane-N, N,N',N'-tetracetic acid [BAPTA].

**16.** A reagent according to claim 14, wherein said selective complexing agent is ethylenebis(oxyethylenenitrilo) tetraacetic acid [EGTA].

**17.** A reagent according to claim 16, further comprising strontium ions.

**18.** A reagent according to any of claims 13 to 17, which is buffered to a pH in the range 7.5 to 9.5, preferably 8.5 to 8.7.

**19.** A reagent according to claim 18 further comprising the buffer tris(hydroxymethyl)amino methane [TRIS].

**20.** A reagent according to any of claims 13 to 19, further comprising a detergent.

**21.** A reagent according to claim 20, wherein said detergent is an amphoteric detergent, preferably Empigen BB-AU.

**22.** A reagent according to claim 20, wherein said detergent is selected from Triton X-100, Triton X-405 and Brij 35.

**23.** A device for use in the colorimetric determination of magnesium ions in fluids, which comprises a solid support or carrier, and a reagent according to any of claims 13 to 22 deposited on or impregnated into said support or carrier.

**24.** A device according to claim 23, wherein said solid support or carrier comprises plastics material film or a porous solid support.

**Patentansprüche**

**1.** Verfahren zur kolorimetrischen Bestimmung von Magnesiumionen in einem Fluidum durch Kontaktieren einer Probe des betreffenden Fluidums mit einem die metallochrome Farbstoffsubstanz 2-(4,5-Dihydroxy-2,7-disulfo-3-naph-thylazo)benzol-Arsonsäure [Arsenazo I] zusammen mit einem selektiven Komplexbildner umfassenden Reagens und Bestimmen der Bildung eines Magnesium-Farbstoff-Komplexes.

**2.** Verfahren nach Anspruch 1, wobei die Bestimmung der Bildung eines Magnesium-Farbstoff-Komplexes durch Messung der Lichtabsorption von 550-580 nm erfolgt.

**3.** Verfahren nach Anspruch 2, wobei die Bestimmung durch Messung der Lichtabsorption bei oder nahe 568 nm erfolgt.

**4.** Verfahren nach einem der Ansprüche 1 bis 3, wobei der selektive Komplexbildner störende Kationen, insbesondere Calciumionen, aus der Fluidumprobe entfernt.

**5.** Verfahren nach Anspruch 4, wobei der selektive Komplexbildner aus 1,2-Bis(2-aminophenoxy)ethan-N,N,N',N'-tetraessigsäure [BAPTA] besteht.

**6.** Verfahren nach Anspruch 4, wobei der selektive Komplexbildner aus Ethylenbis(oxyethylennitrilo)tetraessigsäure [EGTA] besteht.

**7.** Verfahren nach Anspruch 6, wobei das Reagens zusätzlich Strontiumionen enthält.

8. Verfahren nach einem der Ansprüche 1 bis 7, wobei das Reagens auf einen pH-Wert im Bereich von 7,5 bis 9,5, vorzugsweise 8,5 bis 8,7, gepuffert ist.

9. Verfahren nach Anspruch 8, wobei das Reagens den Puffer Tris (hydroxymethyl) aminomethan [TRIS] enthält.

10. Verfahren nach einem der Ansprüche 1 bis 9, wobei das Reagens zusätzlich ein Detergens zum Löslichmachen irgendeines in der Fluidumprobe enthaltenen Proteins enthält.

11. Verfahren nach Anspruch 10, wobei das Detergens aus einem amphoteren Detergens, vorzugsweise Empigen BB-AU, besteht.

12. Verfahren nach Anspruch 10, wobei das Detergens aus Triton X-100, Triton X-405 und Brij 35 ausgewählt ist.

13. Reagens zur kolorimetrischen Bestimmung von Magnesiumionen in Fluiden, umfassend in Kombination die metallochrome Farbstoffsubstanz 2-(4,5-Dihydroxy-2,7-disulfo-3-naphthylazo)benzol-Arsonsäure [Arsenazo I], und einen selektiven Komplexbildner.

14. Reagens nach Anspruch 13, wobei der selektive Komplexbildner störende Kationen, insbesondere Calciumionen, entfernt.

15. Reagens nach Anspruch 14, wobei der selektive Komplexbildner aus 1,2-Bis(2-aminophenoxy)ethan-N,N,N',N'-tetraessigsäure [BAPTA] besteht.

16. Reagens nach Anspruch 14, wobei der selektive Komplexbildner aus Ethylenbis(oxyethylennitrilo)tetraessigsäure [EGTA] besteht.

17. Reagens nach Anspruch 16, welches zusätzlich Strontiumionen enthält.

18. Reagens nach einem der Ansprüche 13 bis 17, welches auf einen pH-Wert im Bereich von 7,5 bis 9,5 , vorzugsweise 8,5 bis 8,7, gepuffert ist.

19. Reagens nach Anspruch 18, welches zusätzlich den Puffer Tris(hydroxymethyl)aminomethan [TRIS] enthält.

20. Reagens nach einem der Ansprüche 13 bis 19, welches zusätzlich ein Detergens enthält.

21. Reagens nach Anspruch 20, wobei das Detergens aus einem amphoteren Detergens, vorzugsweise Empigen BB-AU besteht.

22. Reagens nach Anspruch 20, wobei das Detergens aus Triton X-100, Triton X-405 und Brij 35 ausgewählt ist.

23. Vorrichtung zur Verwendung bei der kolorimetrischen Bestimmung von Magnesiumionen in Fluiden, umfassend einen festen Schichtträger oder Träger und ein Reagens nach einem der Ansprüche 13 bis 17, welches auf dem Schichtträger oder Träger abgelagert oder in diesen imprägniert ist.

24. Vorrichtung nach Anspruch 23, wobei der feste Schichtträger oder Träger aus einem Kunststofffilm oder einem porösen festen Träger besteht.

**Revendications**

1. Procédé pour la détermination colorimétrique d'ions magnésium dans un fluide qui comprend les étapes consistant à mettre en contact un échantillon dudit fluide avec un réactif comprenant une substance colorante métallochrome, acide 2-(4,5-dihydroxy-2,7-disulfo-3-naphthylazo) benzène-arsonique [Arsenoazo I], avec simultanément un agent complexant sélectif et à déterminer la formation d'un complexe colorant-magnésium.

2. Procédé selon la revendication 1 dans lequel ladite détermination de la formation du complexe colorant-magnésium est réalisée par mesure de la lumière d'absorption de 550-580 nm.

3. Procédé selon la revendication 2 dans lequel ladite détermination est réalisée par mesure de la lumière d'absorption à ou proche de 568 nm.

4. Procédé selon l'une quelconque des revendications 1 à 3, dans lequel ledit agent complexant sélectif élimine les cations interférents, en particulier les ions calcium, dudit échantillon de fluide.

5. Procédé selon la revendication 4 dans lequel ledit agent complexant sélectif est l'acide 1,2-bis (2-aminophénoxy) éthane-N,N,N',N'-tétracétique [BAPTA].

6. Procédé selon la revendication 4 dans lequel ledit agent complexant sélectif est l'acide éthylènebis (oxyéthylènenitrilo) tétraacétique [EGTA].

7. Procédé selon la revendication 6 dans lequel le réactif comprend en outre des ions strontium.

8. Procédé selon l'une quelconque des revendications 1 à 7 dans lequel ledit réactif est tamponné à un pH dans le domaine de 7,5 à 9,5 de préférence de 8,5 à 8,7.

9. Procédé selon la revendication 8 dans lequel ledit réactif comprend le tampon tris(hydroxyméthyl) amino méthane [TRIS].

10. Procédé selon l'une quelconque des revendications 1 à 9, dans lequel ledit réactif comprend en outre un détergent pour solubiliser toute protéine présente dans ledit échantillon de fluide.

11. Procédé selon la revendication 10 dans lequel ledit détergent est un détergent amphotère de préférence l'Empigen BB-AU.

12. Procédé selon la revendication 10 dans lequel ledit détergent est choisi parmi le Triton X-100, le Triton X-405 et le Brij 35.

13. Réactif pour la détermination colorimétrique d'ions magnésium dans des fluides qui comprend en combinaison la substance colorante métallochrome, acide 2-(4,5-dihydroxy-2,7-disulfo-3-naphthylazo) benzène-arsonique [Arsenoazo I], avec simultanément un agent complexant sélectif.

14. Réactif selon la revendication 13 dans lequel ledit agent complexant sélectif élimine les cations interférents en particulier les ions calcium.

15. Réactif selon la revendication 14 dans lequel ledit agent complexant sélectif est l'acide 1,2-bis(2-aminophénoxy) éthane-N,N,N',N'-tétracétique [BAPTA].

16. Réactif selon la revendication 14 dans lequel ledit agent complexant sélectif est l'acide éthylènebis (oxyéthylènenitrilo) tétraacétique [EGTA].

17. Réactif selon la revendication 16 qui comprend en outre des ions strontium.

18. Réactif selon l'une quelconque des revendications 13 à 17, qui est tamponné à un pH dans le domaine de 7,5 à 9,5, de préférence de 8,5 à 8,7.

19. Réactif selon la revendication 18 qui comprend en outre le tampon tris(hydroxyméthyl) amino méthane [TRIS].

20. Réactif selon l'une quelconque des revendications 13 à 19 qui comprend en outre un détergent.

21. Réactif selon la revendication 20 dans lequel ledit détergent est un détergent amphotère, de préférence l'Empigen BB-AU.

22. Réactif selon la revendication 20 dans lequel ledit détergent est choisi parmi le Triton X-100, le Triton X-405 et le Brij 35.

23. Dispositif destiné à être utilisé pour la détermination colorimétrique d'ions magnésium dans des fluides, qui comprend un support ou véhicule solide et un réactif selon l'une quelconque des revendications 13 à 22 déposé sur ou imprégné dans ledit support ou véhicule.

24. Dispositif selon la revendication 23 dans lequel le support ou véhicule solide comprend un film en matériau plastique ou un support solide poreux.

FIGURE 1

MAGNESIUM
Arsenazo versus AAS

ARS1 = .90AAS + .16

corr.coeff = .99   n = 21

# MAGNESIUM
## Effect of added calcium.

EP 0 597 900 B1

FIGURE 2

FIGURE 3

ARSENAZO MAGNESIUM
"Open boat" reagent stability

(Ars1) ■ (Ars1+Mg) + (Cal) ✳ (Cal+Mg) □

# ARSENAZO MAGNESIUM
## Absorbance versus time

EP 0 597 900 B1

FIGURE 4

# MAGNESIUM RECOVERY
## Actual Mg vs. theoretical Mg.

EP 0 597 900 B1

FIGURE 5

# ARSENAZO MAGNESIUM
## Absorbance vs. concentration

EP 0 597 900 B1

FIGURE 6